# EUROPEAN PATENT SPECIFICATION

(11) **EP 1 583 522 B1**
(45) Date of publication and mention of the grant of the patent: **08.10.2008**
(21) Application number: 04701143.2
(22) Date of filing: 09.01.2004
(51) Int. Cl.: A61K 31/138, A61P 35/00, A61K 31/00

(54) **USE OF CYCLIN D1 INHIBITORS FOR BREAST CANCER TREATMENT**
VERWENDUNG VON CYCLIN-D1-HEMMERN ZUR BRUSTKREBSBEHANDLUNG
UTILISATION D'INHIBITEURS DE LA CYCLINE D1 POUR LE TRAITEMENT DU CANCER DU SEIN

(30) Priority: 15.01.2003 SE 0300098
(43) Date of publication of application: 12.10.2005
(73) Proprietor: Forskarpatent i Syd AB, 223 70 Lund (SE)
(72) Inventor: KRONBLAD, Asa, S-211 29 Malmö (SE); STENDAHL, Maria, S-553 23 Jönköping (SE); LANDBERG, Göran, S-216 15 Limhamn (SE)
(74) Representative: Inger, Lars Ulf Bosson
(86) International application number: PCT/SE2004/000006
(87) International publication number: WO 2004/062654

(56) References cited:
- WO-A1-00/57875
- BRACKE M.E. ET AL.: 'Influence of Tangeretin on Tamoxifen's Therapeutic Benefit in Mammary Cancer' JOURNAL OF THE NATIONAL CANCER INSTITUTE vol. 91, no. 4, 17 February 1999, pages 354 - 359, XP009002364
- DEPYPERE H.T. ET AL.: 'Inhibition of tamoxifen's therapeutic benefit by tangeretin in mammary cancer' EUROPEAN JOURNAL OF CANCER vol. 36, 2000, page S73, XP002980028
- SUGANUMA M. ET AL.: 'Synergistic Effects of (-)-Epigallocatechin Gallate with (-)-Epicatechin, Sulindac, or Tamoxifen on Cancer-preventice Activity in the Human Lung Cancer Cell Line PC-9' CANCER RESEARCH vol. 59, 01 January 1999, pages 44 - 47, XP000929404
- HUI ET AL.: CANCER RESEARCH, vol. 62, 1 December 2002 (2002-12-01), pages 6916-6923, XP008069152

## Description

### Technical field

The present invention relates to use of certain cyclin D1 inhibitors at the manufacture of pharmaceutical preparations to be used in the treatment of patients to improve their response to tamoxifen treatment following a breast cancer treatment, either surgically, using cytotoxic compounds and/or irradiation.

### Background of the invention

Anti-estrogen treatment by tamoxifen is a well-established adjuvant therapy for estrogen receptor (ER) positive breast cancer. Despite ER-positivity some tumours do not respond to tamoxifen and therefore the potential link between the ER co-factor cyclin D1 and tamoxifen response in 102 ER-positive tumours from post-menopausal breast cancer patients randomised to tamoxifen or no treatment has been delineated.

Thereby it could be noted that patients with moderate cyclin D1 levels responded to tamoxifen treatment whereas patients with cyclin D1 over expressing tumours did not show any difference in survival between tamoxifen and non-tamoxifen treatments. The results suggest that cyclin D1 over expression predicts for treatment resistance but despite this, indicates an overall better prognosis.

Breast cancer is a highly heterogeneous disease that should ideally be subcategorised according to genetic defects potentially mirroring prognostic and predictive information in order to assure optimal and individualised treatment for patients. Adjuvant treatment with anti-estrogens like tamoxifen is one of the most important treatment strategies used for breast cancer, saving many lives. The presence of ER in tumour cells is essential for tamoxifen response and the ER together with the progesterone receptor serves as a predictive factor for tamoxifen response in clinical practise. Despite ER-positivity some tumours do not respond or develop resistance to tamoxifen treatment suggesting that the presence of ER is not the only factor influencing tamoxifen response. Even though the rational for treatment failure is not fully comprehended, cofactors to the ER such as cyclin D1 are suggested to be implicated in this process. Cyclin D1 is a cell cycle regulating protein with potential dual roles and in addition to activating cdk 4/6 in the G1/S transition the protein has cdk-independent functions (1, 2). Some reports propose that cyclin D1 over expression can activate the ER independent of ligand but the theoretically important feature for cyclin D1 in response to tamoxifen treatment has shown contradictory results using breast cancer cell lines (3, 4, 5) and no randomized clinical studies have been reported. Cyclin D1 knockout mice show a marked defect in breast epithelium development during pregnancy and tissue specific over-expression of cyclin D1 leads to mammary hyperplasia and adenocarcinoma formation in mice models, supporting the relevance for cyclin D1 in breast cancer research and its potential as a co-factor for the ER (2).

Cancer Research, vol. 59, (1999), p. 44-47, Suganuma et al relates to a use of tamoxifen in the treatment of lung cancer. However, there is very little of estrogen receptor (ER) present in cancer cells in lung tissue compared to breast cancer cells. Laura Stabile et al, Cancer Res. 62:2141-2150, (2002) have shown that there is much lower levels of ER present in different lung cancer cell lines in comparison with the breast cancer cell line MCF-7. Thus, one cannot expect the same effect of the ER antagonist tamoxifen in a breast cancer cell line as in a lung cancer cell line. Suganuma et al neither show any presence of ER in PC-9 cells used in the trials.

When it comes to breast cancer tamoxifen treatment requires a presence of ER in more than 10 % of the cells.

The doses of tamoxifen used by Suganuma et al is, further, much higher than those used at the inhibition of breast cancer growth in a cell culture, and a clinical evaluation, respectively. Using such high concentrations, as Suganuma et al use, one obtains an evident toxic effect with a high degree of apoptosis, while lower concentrations provides for a specific cell cycle blockade and no apoptosis. In general there is thus different effect mechanisms present in the Suganuma study, compared to the study given herein below, and which are used for a combination therapy using cyclin D1-inhibitors and tamoxifen.

In the same article it is further discussed unpublished data concerning an additive effect of green tea and tamoxifen and it is referred to animal models showing a lower frequency of breast cancer in tamoxifen plus green tea treated mice. However, it is still discussed high, toxic concentrations of tamoxifen, and will not discuss the facts behind the present invention, i.e., that a cofactor of ER, as cyclin D1, may influence the response to tamoxifen.

Hui et al.: Cancer Research, vol.62,1 December 2002, pages 6916-6923 uses randomised untreated or tamoxifen treated patients with a long follow-up period indicating that cyclin D1 affects tamoxifen response.

Suganuma et al.: Cancer Research, vol. 59, 1 January 1999, pages 44-47 47 relates to the synergistic effects of (-)-Epigallocathechin Gallate with (-)-Epicathechin, Sulindac, or Tamoxifen on cancer preventive activity in human lung cancer.

### Description of the present invention

It has turned out that the response to tamoxifen treatment following a breast cancer treatment, either surgical, using cytotoxic compounds and/or irradiation is improved using one or more of the cyclin **D1** inhibitors selected from the group consisting of monoterpenes, nordihydrogualaretic acid, acyclic retinoid (ACR), sesquicillin, sulindac (an NSAID), methylglyoxal bis (cyclopentylamidinohydrazone), ANXA-1, FR-901228 (a cyclic peptide inhibitor of histone deacetylase), simvastatin (mevalonate/protein prenylation inhibitor), cerivastatin (inhibitor of HMG-CoA reductase), (-)-enantiomer of glossypol (polyphenolic pigment present In cottonseed), ursolic acid (pentacyclictrlterpenold), 14-epi-analogues of 1,25-dihydroxyvitamin D3, tangeritin (5,6,7,8,4'-pentamethoxyflavone), purvalanol A (protein kinase inhibitor), tetrandrine, deoxybouvardin, lycopene, podophyllotoxin GL331, resveratrol, silymarin, eplgallocatechin-3-gallate (EGCG), piceatannol, exisulind, oxamflatin, androstanes and androstenes and prostaglandin A2 In the manufacture of a pharmaceutical preparation for the treatment of patients to improve their response to anti-estrogen treatment following a breast cancer treatment, either surgical, using cytotoxic compounds and/or irradiation.

In a preferred embodiment the pharmaceutical preparation is intended for the treatment of tamoxifen none-responsive breast cancer patients over expressing cyclin D1**.**

In another preferred embodiment the pharmaceutical preparation is Intended for the treatment of breast-cancer patients expressing low or moderate levels of cyclin D1**.**

In a preferred embodiment of the Invention the anti-estrogen compound is tamoxifen.

### Study

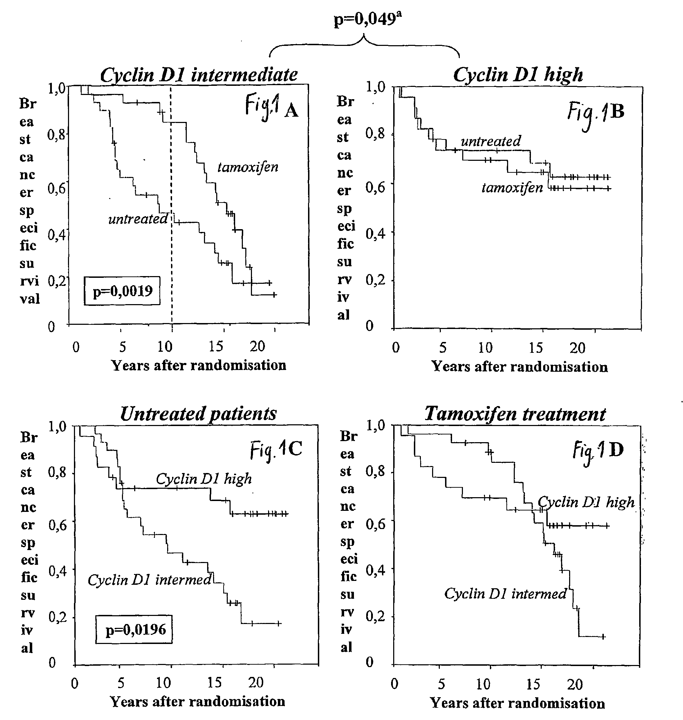

The breast cancer material used In this study Initially included 168 post-menopausal (>55 years old) patients with small (T0-T1) node-negative (NO) tumours. All patients were part of a clinical trial (1980 - 1987) and had been randomised to either 2 years of tamoxifen treatment or no adjuvant treatment. Data regarding breast cancer specific survival was obtained from the Swedish Cancer Registry (2002) resulting In a mean follow-up time of 18 years (range 15-22 years). Representative parts of the tumours were assembled in a tissue array, sectioned and lmmunohistochemically stained for ER (antibodies M7047 from Dako, Denmark, diluted 1/200) and cyclin D1 (M7155, Dako, Denmark, 1/100). Patients with tumours lacking or expressing low ER as well as tumours with 10-90% ER positive cells did not show any significant difference In survival for tamoxifen In comparison to no treatment and were therefore not used for the cyclin D1 studies (data not shown). The 102 tumours with >90% ER positive cells, nevertheless showed a marked difference in survival (p-value) and were selected for further studies. Cyclin D1 protein was evaluated by determining nuclear staining intensity (0-3) and 45% of the 102 ER high tumours were cyclin D1 high-expressing whereas 50% were moderate expressing and 5% were cyclin D1 low expressing. By this definition, around half of the ER positive tumours over-expressed cyclin D1, which is in line with earlier reports (2). As Illustrated In figure 1A there was a marked difference In survival between patients that had received tamoxifen or no treatment when patients with moderate/low cyclin D1 tumours were analysed separately (p = 0.0019 at 10 years). Surprisingly, this difference was eliminated for tumours with high cyclin D1 (figure 1B), suggesting that over expression of cyclin D1 is linked to tamoxifen treatment resistance despite high ER-content. Using multivariate analysis and a Cox-regression model, limiting the follow-up time to 10 years and Including the age at onset of disease, the difference in tamoxifen response between the two cyclin D1 groups were statistically significant (p=0,049) dearly validating the results. There was further a marked difference between the cyclin D1 groups regarding survival for untreated patients with a mortality rate of 35% and 72% respectively for cyclin D1 high contra cyclin D1 moderate/low tumours (table 1). This suggests that high levels of cyclin. D1 are associated with an overall better prognosis than moderate or low cyclin D1 levels as also Illustrated in figure 1C. Interestingly, the opposite was observed, when analysing only patients treated with tamoxifen (figure 1D), which is In line with eariler publications that suggest cyclin D1 over-expression to be associated with bad prognosis.

Our results using randomised untreated or tamoxifen treated patients with a long follow-up period indicate that cyclin D1 indeed affects tamoxifen response and the most likely mechanistic explanation for this is through a direct interaction between cyclin D1 and the ER/SRC, or via its cell cycle regulatory function, as also supported by cell line studies (4, 5). Cyclin D1 could potentially block the effect of tamoxifen on the ER despite theoretically causing an estrogen independent low activation. The alternative model is that cyclin D1 could sequester cdk-inhibitors thereby effecting the G1/S control and tamoxifen response. Further studies now have to verify our results but it seems that a large fraction of patients who receive tamoxifen do not benefit from it. On the other hand they have a rather favourable prognosis. The outcome could nevertheless potentially be improved by specifically targeting cyclin D1 in conjunction with tamoxifen, representing a new treatment strategy for tamoxifen resistance in ER-positive cyclin D1 over expressing breast cancer.

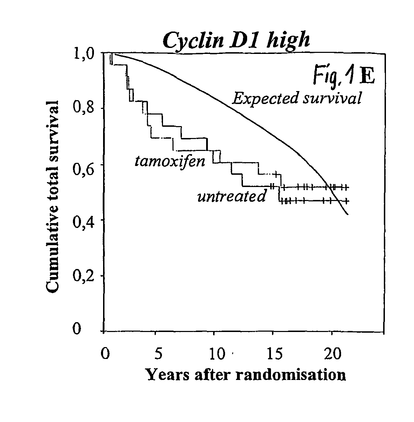

Figure 1 shows the response to tamoxifen treatment of breast cancer patients. Tumours with moderate levels of cyclin D1 expressed show response to tamoxifen treatment but low over all survival (1A). High levels of cyclin D1 expressed predict for treatment resistance but better over all survival (1B). Thus figure 1B shows that there is no real difference between the survival of non-tamoxifen patients and tamoxifen patients with regard to survival, which indicates that the tamoxifen treatment has little or no effect in the high cyclin D1 expressing group.

**Table 1**

| Patient material summarised by subgroups. | | | | |
|---|---|---|---|---|
| Number of patients in different groups studied; mean age; survival time and mortality rates. | | | | |
| | Moderate/low CD1 and no treatment | Moderate/low CD1 and tamoxifen treatment | High CD1 and no treatment | High CD1 and tamoxifen treatment |
| Number of patients | 29 | 27 | 23 | 23 |
| Median age at onset of disease | 70.3 years | 68.8 years | 64.8 years | 63.3 years |
| Median survival time of deceased patients | 65 months | 169 months | 35 months* | 47 months* |
| Mortality rate | 72% | 63% | 35% | 39% |

| | | | | |
|---|---|---|---|---|
| * Variation due to distribution of cases. Mean values near equal. | | | | |

### FIGURE LEGENDS

### Figure 1

A-D: Breast cancer specific survival in relation to cyclin D1 and the randomisation to 2 years of tamoxifen treatment or no treatment in a material of 102 strongly ER-positive breast cancer samples. The p-value in A corresponds to 10 years of follow-up as indicated in the figure.
^{a}Multivariate analyses using a cox-model including age at onset illustrating the significant difference in response to tamoxifen treatment in the cyclin D1 groups.
E: Cumulative total survival in the patient group of cyclin D1 high breast cancer subdivided according to tamoxifen treatment or nor treatment. The calculated expected survival for the specific patient group and year is indicated in the figure.

### REFERENCES

1. Zwijsen RM, Wientjens E, Klompmaker R, van der Sman J, Bernards R, Michalides RJ. CDK-independent activation of estrogen receptor by cyclin D1. Cell 7: 405-415,
2. Zhou Q, Hopp T, Fuqua SA, Steeg PS. Cyclin D1 in breast cancer premalignancy and early breast cancer: implications for prevention and treatment. Cancer Lett 10: 3-17, 2001.
3. Pacilio C, Germano D, Addeo R, Altucci L, Petrizzi VB, Cancemi M, Cicatiello L, Salzano S, Lallemand F, Michalides RJ, Breciani F, Weisz A. Constitutive overexpression of cyclin D1 does not prevent inhibition of hormone-responsive human breast cancer cell growth by antiestrogens. Cancer Res 1: 871-876, 1998.
4. Bindels EMJ, Lallemand F, Balkenende A, Verwoerd D, Michalides R. Involvement of G1/S cyclins in estrogen-independent proliferation of estrogen receptor positive breast cancer cells. Oncogene 21: 8158-8165, 2002.
5. Hui R, Finney GL, Carroll JS, Lee CSL, Musgrove EA, Sutherland RL. Constitutive ocerexpression of cyclin D1 but not Cyclin E confers acute resistance to antiestrogens in T-47D breast cancer cells. Cancer Res 62, 6916-6923, 2002.

## Claims

1. The use of one or more cyclin D1 Inhibitor selected from the group consisting of monoterpenes, nordihydroguaiaretic acid, acyclic retinoid (ACR), sesquicillin, sulindac (an NSAID), methylglyoxal bis (cyclopentylamidinohydrazone), ANXA-1, FR-901228 (a cyclic peptide inhibitor of histone deacetylase), simvastatin (mevalonate/protein prenylation inhibitor), cerivastatin (inhibitor of HMG-CoA reductase), (-)-enantiomer of glossypol (polyphenolic pigment present In cottonseed), ursolic acid (pentacyclictriterpenoid), 14-epi-analogues of 1,25-dihydroxyvitamin D3, tangeritin (5,6,7,8,4'-pentamethoxyflavone), purvalanol A (protein kinase inhibitor), tetrandrine, deoxybouvardin, lycopene, podophyllotoxin GL331, resveratrol, silymarin, epigallocatechin-3-gallate (EGCG), piceatannol, exisulind, oxamflatin, androstanes and androstenes and prostaglandin A2 in the manufacture of a pharmaceutical preparation for the treatment of patients to improve their response to anti-estrogen treatment following a breast cancer treatment, either surgical, using cytotoxic compounds and/or Irradiation.

2. The use according to claim 1, wherein the pharmaceutical preparation is intended for the treatment of anti-estrogen non-responsive breast cancer patients over expressing cyclin D1.

3. The use according to claim 1, wherein the pharmaceutical preparation Is intended for the treatment of breast-cancer patients expressing low or moderate levels of cyclin D1**.**

4. The use according to claims 1-3, wherein the anti-estrogen compound is tamoxifen.

## Patentansprüche

1. Verwendung eines oder mehrerer Cyclin D1-Inhibitoren ausgewählt aus der Gruppe bestehend aus Monoterpenen, Nordihydrogualaretinsäure, Acrylretinoid (ACR), Sesquicillin, Sulindac (einem NSAIM), Methylglyoxal-bis (Cyclopentylamindinohydrazon), ANXA-1, FR-901228 (einem cyclischen Peptidinhibitor von Histondeacetylase), Simvastatin (Mevalonat(Proteinprenylationsinhibitor), Cerivastatin (Inhibitor der HMG-CoA-Reduktase), (-)-Enantiomer von Glossypol (Polyphenolpigment, das in Baumwollsamen vorliegt), Ursolsäure (pentacyclisches Triterpenoid), 14-Epi-Analogen von 1,25-Dihydroxyvitamin D3, Tangeritin (5,6,7,8,4'-Pentamethoxyflavon), Purvalanol A (Proteinkinaseinhibitor), Tetrandrin, Desoxybouvardin, Lycopen, Podophyllotoxin GL331, Resveratrol, Silymarin, Epigallocatechin-3-gallat (EGCG), Piceatannol, Exisulind, Oxamflatin, Androstane und Androstene und Prostaglandin A2 bei der Herstellung einer pharmazeutisch Zubereitung für die Behandlung von Patienten zum Verbessern ihrer Reaktion auf die Antiöstrogenbehandlung auf eine Brustkrebsbehandlung entweder durch Chirurgie, unter Anwendung zytotoxischer Verbindungen und/oder von Bestrahlung hin.

2. Verwendung nach Anspruch 1, wobei die pharmazeutische Zubereitung der Behandlung von auf Antiöstrogen nicht ansprechenden Brustkrebspatienten, die Cyclin D 1 überexprimieren, dienen soll.

3. Verwendung nach Anspruch 1, wobei die pharmazeutische Zubereitung der Behandlung von Brustkrebspatienten, die geringe oder mäßige Niveaus an Cyclin D1 exprimieren, dienen soll.

4. Verwendung nach den Ansprüchen 1-3, wobei die Antiöstrogenverbindung Tamoxifen ist.

## Revendications

1. Utilisation d'un ou plusieurs inhibiteurs de la cycline D1 choisis dans le groupe consistant en monoterpènes, acide nordihydroguaiarétique, rétinoïde acyclique (ACR), sesquicilline, sulindac (un NSAID), méthylglyoxal bis (cyclopentylamidinohydrazone), ANXA-1, FR-901228 (un peptide cyclique inhibiteur de l'histone desacétylase), simvastatine (mévalonate/inhibiteur de la prénylation des protéines), cérivastatine (inhibiteur de HMG-CoA réductase), énantiomère-(-) de glossypol (pigment polyphénolique présent dans les graines de coton), acide ursolique (triterpénoïde pentacyclique), 14-épi-analogues de 1,25-dihydroxyvitamine D3, tangéritine (5,6,7,8,4'-pentaméthoxyflavone), purvalanol A (inhibiteur de la protéine kinase), tétrandrine, désoxybouvardine, lycopène, podophyllotoxine GL331, resvératrol, silymarine, épigallocatéchine-3-gallate (EGCG), picéatannol, exisulinde, oxamflatine, androstanes et androstènes et prostaglandine A2 dans la fabrication d'une préparation pharmaceutique destinée au traitement de patientes pour améliorer leur réponse au traitement anti-oestrogène consécutif à un traitement du cancer du sein, que ce traitement se fasse par voie chirurgicale, en utilisant des composés cytotoxiques, et/ou par irradiation.

2. Utilisation selon la revendication 1, dans laquelle la préparation pharmaceutique est destinée au traitement de patientes atteintes d'un cancer du sein qui sont non sensibles au traitement anti-oestrogène et qui surexpriment la cycline D1.

3. Utilisation selon la revendication 1, dans laquelle la préparation pharmaceutique est destinée au traitement de patientes atteintes de cancer du sein exprimant des niveaux faibles ou modérés de cycline D1.

4. Utilisation selon les revendications 1-3, dans laquelle le composé anti-oestrogène est le tamoxifène.
